# EUROPEAN PATENT APPLICATION

(11) **EP 3 293 170 A1**
(43) Date of publication of application: **14.03.2018**
(21) Application number: 16789569.7
(22) Date of filing: 27.04.2016
(51) Int. Cl.: C07C 17/25, C07C 17/383, C07C 21/04, C07B 61/00

(54) **CHLOROALKENE PRODUCTION METHOD ACCOMPANIED WITH DEHYDROCHLORINATION REACTION**

(30) Priority: 07.05.2015 JP 2015094724
(71) Applicant: Tokuyama Corporation, Shunan-shi, Yamaguchi 745-0053 (JP)
(72) Inventor: HOSAKA, Syunsuke, Shunan-shi Yamaguchi 745-0053 (JP); NISHIMOTO, Ryousuke, Shunan-shi Yamaguchi 745-0053 (JP)
(74) Representative: Beckmann, Claus
(86) International application number: PCT/JP2016/063794
(87) International publication number: WO 2016/178433

(57) **Abstract**

A chloroalkene production method comprising the steps of:
carrying out the dehydrochlorination reaction of a chloroalkane in the presence of a Lewis acid catalyst containing a metal chloride to produce a chloroalkene;
removing a metal component derived from the catalyst from the obtained reaction solution; and
distilling the reaction solution to obtain the purified chloroalkene.

## Description

### TECHNICAL FIELD

The present invention relates to a chloroalkene production method accompanied by a dehydrochlorination reaction. More specifically, it relates to a method of obtaining a purified chloroalkene by distillation after a chloroalkane is subjected to a dehydrochlorination reaction using a metal chloride as a catalyst.

### BACKGROUND ART

Chloroalkenes are important as raw materials or intermediates for the manufacture of products such as agricultural chemicals, medicines, chlorofluorocarbon-replacing materials and fluorine compounds. For example, 1,1,2,3-tetrachloropropene is known as a raw material for producing trichloroallyl diisopropylthiocarbamate which is useful as a herbicide.

As the method of producing these chloroalkenes, there is known a two-stage reaction consisting of a first reaction for obtaining a chloropropane by adding carbon tetrachloride to an unsaturated compound having 2 carbon atoms (non-substituted or chlorine-substituted ethylene) and a second reaction for obtaining a chloropropene by dehydrochlorinating the chloropropane. Stated more specifically, JP-B 2-47969 discloses a method in which ethylene is reacted with carbon tetrachloride in the presence of a catalyst containing metal iron and a trialkyl phosphate to obtain 1,1,1,3-tetrachloropropane and then 1,1,1,3-tetrachloropropane is dehydrochlorinated to obtain 1,1,3-trichloro-1-propene or 3,3,3-trichloro-1-propene. JP-A 2008-531474 discloses a method of obtaining 1,1,3,3-tetrachloro-1-propene by reacting carbon tetrachloride with vinyl chloride in the presence of a metal chloride containing metal iron and tributyl phosphate as a catalyst to obtain 1,1,1,3,3-pentachloropropane and subjecting it to a dehydrochlorination reaction in the presence of a Lewis acid catalyst.

JP-B 2011-507877 discloses a method of producing 1,1,2,3-tetrachloro-1-propene by carrying out a dehydrochlorination reaction using iron chloride as a catalyst after 1,1,1,2,3-pentachloropropane is obtained by blowing a chlorine gas in the system while the dehydrochlorination of 1,1,1,3-tetrachloropropane is carried out by using iron chloride as a catalyst.

By the way, in general, the polychloroalkene obtained by the second reaction is an intermediate of the above-described raw material and supplied to the subsequent chlorination or fluorination step to be changed to a polychloroalkane, fluorochloroalkane or fluorochloroalkene. The obtained polychloroalkane is generally distilled to improve its purity before it is supplied to the subsequent step. It is known that when the polychloroalkane is supplied to the subsequent step while iron chloride which is the catalyst for the second reaction remains, the dehydrochlorination reaction of a product of interest obtained by chlorination or fluorination proceeds, thereby causing the reduction of yield, so that the above iron chloride should be removed by the above distillation.

However, when distillation is carried out at a high temperature for a long time, a side reaction such as a polymerization reaction or dehydrochlorination reaction proceeds, thereby causing problems such as the reduction of yield and the corrosion of equipment.

Therefore, to prevent the above problems caused by the side reaction when purification by distillation is carried out, a measure such as distillation at a low temperature and a low pressure is taken.

The dehydrochlorination reaction which is caused by the catalyst residue in the second reaction can be prevented by deactivating the above catalyst containing an iron complex. For example, JP-B 2004-534060 teaches that the above dehydrochlorination reaction in the distillation step is suppressed even in the presence of iron (III) such as iron chloride by adding a phenol compound to the polychloroalkane, thereby making it possible to carry out distillation without causing the reduction of yield by the side reaction.

Due to recent growing demand for chloroalkenes, it is becoming necessary to set a high distillation temperature or a low distillation pressure or to carry out distillation continuously for a long time in order to improve production efficiency. If a high distillation temperature is set, when a catalyst is deactivated by adding a phenol compound as described above, it is possible to suppress a dehydrochlorination reaction at the time of distillation caused by the residual catalyst or the dehydrochlorination reaction of a product of interest obtained by chlorination or fluorination in the subsequent step, and further a problem such as the corrosion of equipment caused by the produced hydrogen chloride is solved. However, when distillation is carried out continuously at a high temperature for a long time, there occur new significant problems such as the reduction of yield caused by the polymerization reaction of the chloroalkene and the blockage of a distillation system by the adhesion of a highly viscous material to the inner walls of equipment and pipes.

### DISCLOSURE OF THE INVENTION

It is therefore an object of the present invention to provide a chloroalkene production method capable of carrying out distillation continuously and stably for a long time by reducing the amount of a highly viscous material adhered to the inner walls of equipment and pipes at the time of distillation when a chloroalkene is produced by dehydrochlorinating a chloroalkane in the presence of a Lewis acid catalyst containing a metal chloride and purifying the obtained reaction solution by distillation.

The inventors of the present invention conducted intensive studies to solve the above problem and found that the above highly viscous material causing the blockage of the distillation system is produced not only when iron (III) is used as a catalyst but also when a metal chloride is used as a catalyst, thereby causing the same problem. They also found that the polymerization reaction of the chloroalkene proceeds by long-time operation at a high temperature to highly concentrate a material having a higher boiling point than that of the chloroalkene (to be referred to as "high-boiling point material" hereinafter), such as a dimer or polymer, in the residual solution, which is one of the causes of producing the highly viscous material, and that this highly concentrated high-boiling point material forms an agglomerated body together with a metal component derived from the catalyst to produce a highly viscous material which is adhered to the inner walls of equipment and pipes to block them.

Then, they found that, by carrying out distillation after the metal component derived from the catalyst used in the reaction is removed after the dehydrochlorination reaction, the reduction of yield caused by the dehydrochlorination reaction is eliminated even when a high distillation temperature is kept, the polymerization reaction of the chloroalkene proceeds slowly even when continuous operation is carried out at a high temperature for a long time, and the amount of a material adhered to the equipment at the time of distillation is reduced even when the polymerization reaction proceeds, thereby making it possible to carry out distillation stably without causing the blockage of pipes. The present invention was accomplished based on this finding.

That is, the present invention is a chloroalkene production method comprising the steps of:
carrying out the dehydrochlorination reaction of a chloroalkane represented by the following formula (1) in the presence of a Lewis acid catalyst containing a metal chloride to produce a chloroalkene represented by the following formula (2);
removing a metal component derived from the catalyst from the obtained reaction solution; and
distilling the reaction solution to obtain the chloroalkene.

   Cₐ₊₁H_{b}Cl_{c+4} (1)

   ("a" is an integer of 2 to 4, "b" is an integer of 1 to 2a, and "c" is an integer of 0 to (2a-1), with the proviso that (b+c) is 2a.)

   Cₐ₊₁H_{b-1}Cl_{c+3} (2)

   ("a", "b" and "c" are as defined in the above formula (1).)

### BEST MODE FOR CARRYING OUT THE INVENTION

The chloroalkene production method of the present invention will be described in detail hereinunder. The present invention is a chloroalkene production method comprising the steps of:
carrying out the dehydrochlorination reaction of a chloroalkane represented by the following formula (1) in the presence of a Lewis acid catalyst containing a metal chloride to produce a chloroalkene represented by the following formula (2); and
distilling the obtained reaction solution to obtain the chloroalkene.

   Cₐ₊₁H_{b}Cl_{c+4} (1)

   ("a" is an integer of 2 to 4, "b" is an integer of 1 to 2a, and "c" is an integer of 0 to (2a-1), with the proviso that (b+c) is 2a.)

   Cₐ₊₁H_{b-1}Cl_{c+3} (2)

   ("a", "b" and "c" are as defined in the above formula (1).)

In the present invention, the above method has a big feature that, after the metal component derived from the catalyst used in the above reaction is removed from the obtained reaction solution after the dehydrochlorination reaction, the reaction solution is distilled. The above production method will be described in detail hereinunder.

The chloroalkene represented by the above formula (2) produced by the present invention is a linear chloroalkene having 3 to 5 carbon atoms and 3 to 7 chlorine atoms. Specific examples of the chloroalkene include 1,2,3-trichloro-1-propene, 1,1,3-trichloro-1-propene, 1,1,2-trichloro-1-propene, 2,3,3-trichloro-1-propene, 1,1,2,3-tetrachloro-1-propene, 1,1,3,3-tetrachloro-1-propene, 1,2,3,3,3-pentachloro-1-propene, hexachloropropene, 1,1,3-trichloro-1-butene, 1,1,3,3-tetrachloro-1-butene, 1,1,4,4,4-pentachloro-1-butene, 1,1,3,4,4,4-hexachloro-1-butene, 1,1,5-trichloro-1-pentene, 3,3,5-trichloro-1-pentene and 1,1,3,5,5-pentachloro-1-pentene. Out of the chloroalkenes represented by the above formula (2), chloroalkenes having a boiling point at 101.3 kPa of 130°C or higher are preferred as they can achieve the effect of the present invention efficiently, and 1,1,3-trichloro-1-propene and 1,1,2,3-tetrachloro-1-propene are particularly preferred as they are industrially useful compounds.

In the present invention, the chloroalkane represented by the above formula (1) which is a raw material of the chloroalkene is suitably selected according to a chloroalkene of interest. That is, the chloroalkane should be subjected to a dehydrochlorination reaction to produce the chloroalkene of interest represented by the above formula (2) and is represented by the following formula (1).

Cₐ₊₁H_{b}Cl_{c+4} (1)

In the above formula, "a" is an integer of 2 to 4, "b" is an integer of 1 to 2a, and "c" is an integer of 0 to (2a-1), with the proviso that (b+c) is 2a.

Examples of the chloroalkane include 1,1,1,3-tetrachloropropane, 1,1,2,3-tetrachloropropane, 1,1,2,2-tetrachloropropane, 1,1,1,2,3-pentachloropropane, 1,1,1,2,2-pentachloropropane, 1,1,2,2,3-pentachloropropane, 1,1,1,2,2,3,3-heptachloropropane, 1,1,1,3-tetrachlorobutane, 1,1,1,3,3-pentachlorobutane, 1,1,1,4,4,4-hexachlorobutane, 1,1,1,2,4,4,4-heptachlorobutane, 1,1,1,5-tetrachloropentane, 1,3,3,5-tetrachloropentane and 1,1,1,3,5,5-hexachloropentane. When the chloroalkene represented by the above formula (2) is 1,1,3-trichloro-1-propene, 1,1,1,3-tetrachloropropane is particularly preferably selected and when the chloroalkene represented by the above formula (2) is 1,1,2,3-tetrachloro-1-propene, 1,1,1,2,3-pentachloropropane is particularly preferably selected.

The dehydrochlorination reaction of the chloroalkane is a reaction which proceeds in the presence of a catalyst. As the catalyst, a Lewis acid catalyst containing a metal chloride is used. Examples of the above metal chloride which functions as the Lewis acid catalyst include anhydrous aluminum chloride, anhydrous ferric chloride and lead chloride. Out of these, anhydrous aluminum chloride is preferred as it is a catalyst having high activity for the dehydrochlorination reaction.

The amount of the catalyst in use is preferably 2.0 x 10⁻⁵ to 2.0 x 10⁻² mole, more preferably 5.0 x 10⁻⁵ to 1.0 x 10⁻³ mole based on 1 mole of the chloroalkane used in the reaction. When the amount of the catalyst is too large, the dimerization or polymerization of the chloroalkene represented by the above formula (2) proceeds during the dehydrochlorination reaction, whereby the selectivity of the chloroalkene tends to lower and when the amount is too small, the reaction rate of the dehydrochlorination reaction tends to become low.

The reaction temperature is suitably set according to the type of the catalyst in use, the type of the chloroalkane as the raw material and the type of the chloroalkene of interest and is not particularly limited if it is not higher than the boiling point of the chloroalkene represented by the formula (2). For example, it is preferably 20 to 180°C, more preferably 30 to 150°C, much more preferably 50 to 120°C.

When the reaction temperature is too high for the type of the catalyst in use and the type of the chloroalkane as the raw material, the reactivity of the obtained chloroalkene rises, whereby a by-product is produced by dimerization or a further reaction, and the selectivity of the chloroalkene of interest tends to lower. When the temperature is too low, the dissolution of the catalyst takes long and the reaction rate of the dehydrochlorination reaction tends to become low. When a reaction is carried out at a temperature close to the boiling point of the chloroalkene of interest, a gas phase part is preferably cooled and refluxed in consideration of the evaporation of the chloroalkene.

The metal chloride as the catalyst may be supplied into the reactor before or after the supply of the chloroalkane, and a predetermined amount of the metal chloride may be supplied totally at a time at the beginning, or in batches or continuously during the reaction regardless of a batch reaction or a continuous reaction. Further, the metal chloride which is the catalyst may be dissolved in the chloroalkane outside the reactor and supplied into the reactor as a solution.

The reaction time which differs according to the size of the reactor, the charge-in quantity and the reaction temperature is preferably 30 minutes to 12 hours, more preferably 1 to 6 hours. When the reaction is carried out continuously, the residence time is preferably 30 minutes to 12 hours, more preferably 1 to 6 hours. When it is too short, the conversion rate of the chloroalkane becomes low and when it is too long, a side reaction proceeds and the selectivity of the chloroalkene becomes low.

For example, when anhydrous aluminum chloride is used as the catalyst and 1,1,1,2,3-pentachloropropane is used as the raw material chloroalkane to obtain 1,1,2,3-tetrachloro-1-propene and the reaction temperature is set to 50 to 120°C, the reaction time is about 1 to 6 hours. When anhydrous aluminum chloride is used as the catalyst and 1,1,1,3-tetrachloropropane is used as the raw material chloroalkane to obtain 1,1,3-trichloro-1-propene and the reaction temperature is set to 30 to 80°C, the reaction time is about 1 to 6 hours.

Then, the metal component derived from the catalyst which is contained in the reaction solution obtained by the above-described reaction is removed, which is the most characteristic feature of the present invention. In the present invention, the metal component derived from the catalyst includes a metal chloride, a metal hydroxide and a metal complex which is produced by reacting the metal chloride or the metal hydroxide with another component contained in the reaction solution.

Although the method of removing the above metal component is not particularly limited, an extraction method and an adsorption method may be employed. The extraction method which can remove the metal component effectively and is inexpensive is preferably used. The extraction method is a method in which two solvents not miscible with each other are used to remove the metal component by making use of a difference in metal component solubility between these solvents and called "liquid-liquid extraction method" or "solvent extraction method".

A description is subsequently given of the extraction method for removing the metal component. The types of solvents used for extraction are not particularly limited if they can be separated from the chloroalkene represented by the above formula (2) to form two phases and their solubilities for the metal component to be removed are higher than that of the chloroalkene represented by the formula (2) . Examples of the solvents include water and polyhydric alcohols. Out of these, water, ethylene glycol, propylene glycol, diethylene glycol and glycerin, all of which have low affinity for chloroalkenes, are preferably used.

The amount of each of the extraction solvents used in the above extraction method is preferably 0.01 to 1 part by mass, more preferably 0.03 to 0.3 part by mass, particularly preferably 0.04 to 0.1 part by weight based on 1 part by mass of the chloroalkene represented by the above formula (2). When the amount of the extraction solvent exceeds the above range, its effect hits the ceiling and its cost becomes high. When the amount falls below the above range, it is difficult to separate the chloroalkene and remove the metal component efficiently. After extraction, the extraction solvents in use may be recycled as extraction solvents again after the metal component is removed by using purification means such as distillation after extraction.

When water is used as the above extraction solvent, the pH of water after extraction is preferably 4 or less, more preferably 3 or less. When the pH is at a neutral range from 4 to 7, the metal chloride reacts with a hydroxide ion to become a metal hydroxide according to the type of the metal of the metal chloride in use. Metal hydroxides have very low solubility in a solvent as compared with metal chlorides in most cases and tend to precipitate as a solid. When the metal hydroxide precipitates as a solid, it is difficult to carry out extraction with a solvent. Therefore, to prevent the formation of the metal hydroxide, the pH of the extraction solvent is preferably at an acidic range. Even when a polyhydric alcohol is used as the extraction solvent, if it contains water like ethylene glycol, the metal chloride tends to become a metal hydroxide as well. Therefore, it is preferred to prevent the formation of the metal hydroxide likewise. In the case of a polyhydric alcohol, the concentration of hydrogen chloride after extraction is preferably not less than 1.0 x 10⁻⁵ part by mass, more preferably not less than 1.0 x 10⁻⁴ part by mass based on 1 part by mass of the polyhydric alcohol.

Therefore, the concentration of hydrogen chloride contained in the reaction solution to be supplied for extraction is preferably 1.0 x 10⁻⁵ to 5.0 x 10⁻² part by mass, more preferably 1.0 x 10⁻⁴ to 1.0 x 10⁻² part by mass, particularly preferably 5.0 x 10⁻⁴ to 5.0 x 10⁻³ part by mass based on 1 part by mass of the chloroalkene of the above formula (2). The precipitation of the metal hydroxide can be prevented by dissolving hydrogen chloride in the reaction solution to be supplied for extraction in the above concentration range.

By the way, the above-described dehydrochlorination reaction is a reaction in which 1 mole of hydrogen chloride is produced when 1 mole of the chloroalkene is produced from the chloroalkane as the raw material. Although a treatment such as the addition of an acid may be carried out to ensure the above range, the precipitation of the metal hydroxide can be easily suppressed by making hydrogen chloride by-produced by the above dehydrochlorination reaction remain in the reaction solution without carrying out the above treatment. Stated more specifically, the above preferred amount of hydrogen chloride is preferably caused to remain without removing all the hydrogen chloride by-produced from the reaction solution by nitrogen substitution operation after the dehydrochlorination reaction. In the present invention, the solution after the reaction contains a large amount of hydrogen chloride and is almost free from the precipitation of the metal hydroxide during extraction. Since the above hydrogen chloride has higher solubility in an extraction solvent than the chloroalkene, it is moved from the chloroalkene to the extraction solvent by the extraction of the present invention. As a result, almost no hydrogen chloride remains in the reaction solution from which the metal component derived from the catalyst supplied into a distillation column has been removed. If hydrogen chloride remains, the extraction method or the adsorption method may be repeated to remove the hydrogen chloride.

The concentration of the metal contained in the reaction solution after the metal component has been removed is preferably not more than 5.0 x 10⁻⁶ part by mass, more preferably not more than 1.0 x 10⁻⁶ part by mass based on 1 part by mass of the chloroalkene of the above formula (2).

Although the extraction method is not particularly limited, a one-stage extraction method, a multi-stage extraction method or a continuous counter-current method may be employed. A suitable method should be selected to ensure that the concentration of the metal contained in the reaction solution after the metal component has been removed falls within the above range.

The obtained reaction solution after the metal component has been removed is distilled, and the chloroalkane and the by-product which are unreacted raw materials are separated and removed to obtain the chloroalkene having high purity and represented by the above formula (2). Since the reaction solution to be distilled has a reduced concentration of the metal component, the dehydrochlorination reaction hardly occurs during distillation and further, even when the high-boiling point material contained in the residual solution is highly concentrated at the bottom of the distillation column having a high temperature, the amount of a material adhered to the distillation column can be reduced, thereby making it possible to carry out distillation continuously and stably at a high temperature for a long time.

In the distillation step of the present invention, the high-purity chloroalkene should be obtained, and distillation for the removal of the high-boiling point material for removing a material having a higher boiling point than that of the chloroalkene and distillation for the removal of a low-boiling point material having a lower boiling point than that of the chloroalkene (to be referred to as "low-boiling point material" hereinafter) may be carried out. In general, only distillation for the removal of the high-boiling point material suffices. The metal component derived from the above-described catalyst is a high-boiling point material and continues to remain in the residual solution at a high temperature during distillation for the removal of the high-boiling point material. Therefore, the effect of the present invention is especially obtained in distillation for the removal of the high-boiling point material. In general, in the above-described reaction for producing the chloroalkene represented by the formula (2) by the dehydrochlorination reaction of the chloroalkane, a by-product having a lower boiling point than that of the chloroalkene represented by the formula (2) is rarely produced.

However, when the metal component is removed by solvent extraction in the step of removing the metal component, according to the type of the solvent used for extraction and extraction operation, a compound having a lower boiling point than that of the chloroalkene represented by the formula (2) may remain in the reaction solution. Therefore, the above distillation for the removal of the high-boiling point material and the above distillation for the removal of the low-boiling point material can be used in combination in consideration of the liquid composition of the reaction solution to be distilled and the desired purity of the chloroalkene. When distillation for the removal of the low-boiling point material is carried out in addition to distillation for the removal of the high-boiling point material, the order of these distillations does not matter but distillation for the removal of the high-boiling point material is preferably carried out after distillation for the removal of the low-boiling point material from the viewpoints of thermal efficiency and the quality of the obtained chloroalkene. When distillation for the removal of the low-boiling point material is to be carried out, the reaction solution from which the metal component has been removed is distilled to remove a material having a lower boiling point than that of the chloroalkene represented by the formula (2) from the top of the column as a distillate and collect the chloroalkene as a solution at the bottom of the column (residual solution).

In the present invention, distillation for the removal of the high-boiling point material is carried out as follows. When distillation for the removal of the low-boiling point material is not carried out, the reaction solution from which the metal component has been removed is subjected to distillation for the removal of the high-boiling point material and when the distillation for the removal of the low-boiling point material is carried out, the solution collected from the bottom of the column is subjected to distillation for the removal of the high-boiling point material to collect the chloroalkene represented by the formula (2) from the top of the column as a distillate. Meanwhile, materials having a higher boiling point than that of the chloroalkene, for example, the chloroalkane as the raw material, a dimer or polymer of the chloroalkene represented by the formula (2) and a metal component which could not be removed in the step of removing the metal component are removed from the solution remaining at the bottom of the column.

In the present invention, any distillation column which is known in the industry may be used as the distillation column used for distillation, and a plate column or packed column is preferably used. For the above plate column, for example, a crossflow tray or shower tray may be used. When the above packed column is used, a known packing material such as Raschig ring or lessing ring may be used. One distillation column or several distillation columns may be used.

The material of the distillation column is not particularly limited and may be a metal, glass or resin. There is a case where water or a small amount of hydrogen chloride is contained in the reaction solution from which the metal component has been removed. Therefore, it is preferred to choose a corrosion-resistant material such as glass, resin, Hastelloy or titanium as the material of a first distillation column. When several columns are used for distillation, hydrogen chloride and water are removed in the first column. The material of a distillation column after hydrogen chloride and water have been removed is not particularly limited.

The material of the packing material is not particularly limited and may be a metal, glass or resin. Since there is a case where hydrogen chloride or water is contained in the reaction solution to be supplied into the distillation column like the material of the above distillation column, it is preferred to choose a corrosion-resistant material such as glass, resin, Hastelloy or titanium. The material of the packing material used in a distillation column after hydrogen chloride and water are removed is not particularly limited as well.

The purity of the chloroalkene obtained by distillation is preferably not less than 98.0 %, more preferably not less than 99.0 %, much more preferably not less than 99.5 %. Although the distillation operation is not particularly limited, the temperature at the top of the column, the temperature at the bottom of the column, the inside pressure of the column, the reflux ratio and the number of stages should be adjusted to ensure that the purity of the chloroalkene falls within the above range. In the present invention, the purity of the above chloroalkene is a value obtained by separating a distillate obtained from the distillation column by gas chromatography (GC) and determining with a hydrogen flame ionizing type detector (FID) . The quantity of a component which cannot be detected with the above hydrogen flame ionizing type detector, for example, water can be determined with a Karl Fischer moisture titrate.

In the distillation operation for separating and removing the low-boiling point material, the temperature range at the bottom of the column is determined in consideration of the type of the chloroalkene of interest, the type of the low-boiling point material, composition and pressure. For example, it is preferably 50 to 180°C, more preferably 80 to 160°C, much more preferably 100 to 150°C.

In the subsequent distillation operation for separating and removing the high-boiling point material, the temperature range at the bottom of the column is determined in consideration of the type of the chloroalkene of interest, the type of the high-boiling point material, composition and pressure as well. For example, it is preferably 70 to 250°C, more preferably 80 to 200°C, much more preferably 100 to 180°C, particularly preferably 120 to 160°C. The above temperature at the bottom of the column refers to the temperature of the solution remaining at the bottom of the column, that is, the solution containing the concentrated high-boiling point material. When the temperature at the bottom of the column exceeds the above range, the chloroalkene and other impurities tend to decompose, resulting in the reduction of yield. When the temperature falls below the above range, it is necessary to cool the top of the column in order to make the temperature at the top of the distillation lower than the temperature at the bottom of the column, thereby increasing the amount of energy required for cooling, which is not efficient. Further, it is necessary to reduce the pressure at the top of the column and the inside pressure of the column, thereby boosting equipment cost and operation cost, which is not efficient as well. In the distillation of the present invention, when the solution is taken out from the distillation column, heated outside and returned to the distillation column, the outside part for heating can be regarded as part of the distillation column, that is, the bottom of the distillation column.

In the present invention, when the temperature at the bottom of the column is set to the above range, the pressure at the time of distillation should be a value which ensures that the chloroalkene represented by the formula (2) vaporizes and reaches the top of the distillation column. Although it is determined according to the type of the chloroalkane, the temperature at the top of the distillation column and the type and size of the distillation column in use, in consideration of equipment cost and operation cost, the pressure at the top of the column is preferably 1 kPa (abs) to 110 kPa (abs), more preferably 5 kPa (abs) to 50 kPa (abs).

The temperature at the top of the column should be a value which ensures that the chloroalkene to be collected reaches the top of the column as a gas under the above pressure and that the chloroalkene can be cooled properly without being discharged to the outside of the distillation column. Therefore, it is determined in consideration of pressure at the time of distillation, the type of the chloroalkene and the boiling point of the high-boiling point material contained in the residual solution. For example, when the chloroalkene represented by the formula (2) is 1,1,2,3-tetrachloro-l-propene, 1,1,2,3-tetrachloro-1-propene can be collected at a high yield with almost no decomposition and side reaction by setting the temperature at the bottom of the column to 120 to 140°C, the temperature at the top of the column to about 95°C and the pressure at the top of the column during distillation to 10 kPa (abs).

When the chloroalkene represented by the formula (2) is 1,1,3-trichloro-1-propene, 1,1,3-trichloro-1-propene can be collected at a high yield with almost no decomposition and side reaction by setting the temperature at the bottom of the column to 100 to 120°C, the temperature at the top of the column to about 71°C and the pressure at the top of the column during distillation to 10 kPa (abs).

Although additives may not be added at the time of distillation, a stabilizer may be added to suppress the thermal decomposition of the chloroalkene of interest. The stabilizer is selected from phenols, for example, phenols substituted by an alkoxy group and phenols substituted by an allyl group. Examples of the phenols substituted by an allyl group include o-allylphenol, m-allylphenol, p-allylphenol, 4-allyl-2-methoxyphenol (eugenol) and 2-methoxy-4-(1-propenyl)phenol (isoeugenol). These allyl-substituted phenols may be used alone or in combination. The amount of the stabilizer to be added is preferably 1.0 x 10⁻⁶ to 2.0 x 10⁻⁴ part by mass, more preferably 1.0 x 10⁻⁶ to 2.0 x 10⁻⁵ part by mass based on 1 part by mass of the chloroalkene represented by the formula (2) contained in the reaction solution to be distilled.

In the distillation step, when a phenol is added as the stabilizer at the time of distillation, it is preferably a phenol having a higher boiling point than that of the chloroalkene represented by the formula (2). When it is so, the decomposition of the chloroalkene can be prevented efficiently by carrying out distillation for separating and removing the high-boiling point material at the end of the distillation step.

The distillation system is not particularly limited. Batch system or continuous system may be used without restriction. According to the present invention, even when the temperature at the bottom of the column is kept high and the residual solution which contains the high-boiling point material is highly concentrated, continuous operation can be carried out stably for a long time. Therefore, a remarkable effect is obtained by carrying out the continuous system.

Although the concentration of the high-boiling point material at the bottom of the column is not particularly limited, the distillation for the removal of the high-boiling point material of the present invention is preferably carried out to ensure that the content of the chloroalkene represented by the formula (2) in the residual solution becomes 0.01 to 3 parts by mass, more preferably 0.05 to 2 parts by mass, much more preferably 0.1 to 1 part by mass based on 1 part by mass of the high-boiling point material.

When the extraction weight of the chloroalkene represented by the formula (2) contained in the material to be removed exceeds the above range, the yield of the chloroalkene of interest drops, thereby reducing production efficiency. When the extraction weight of the chloroalkene represented by the formula (2) contained in the material to be removed falls below the above range, the viscosity of the residual solution increases, thereby making it difficult to remove it from the distillation column.

When a plate column is used, a solution to be extracted each stage for removing part of the high-boiling point material may be added to the residual solution in distillation for the removal of the high-boiling point material.

The chloroalkene obtained as described above is supplied to a chlorination step or fluorination step so as to be used as a raw material or intermediate for the manufacture of products such as agricultural chemicals, medicines, chlorofluorocarbon-replacing materials and fluorine compounds. For example, 1,1,2,3-tetrachloropropene is used as a raw material for the production of trichloroallyl diisopropylthiocarbamate which is useful as a herbicide or a raw material of 2-chloro-3,3,3-trifluoro-1-propene.

### EXAMPLES

The following examples are provided for the purpose of further illustrating the present invention but are in no way to be taken as limiting.

### Example 1

1,421 g of purified 1,1,1,2,3-pentachloropropane having a purity of 99.5 wt% was fed to a 1,000 ml four-necked eggplant flask, and then 0.53 g of anhydrous aluminum chloride was fed to the flask. They were stirred at a liquid temperature of 25°C and then heated by using an oil bath after it was confirmed that the solution turned blue and aluminum chloride was dissolved. Bubbles were produced at 60 to 70°C, and a dehydrochlorination reaction became violent. After the solution temperature was raised to 95°C over 1 hour, the reaction was caused to proceed by keeping the temperature at 95 to 100°C for 1 hour. When the obtained reaction solution was analyzed by GC, the conversion rate of 1,1,1,2,3-pentachloropropane was 96 % and the selectivity of 1,1,2,3-tetrachloro-1-propene was 98 %.

After the obtained reaction solution was cooled to normal temperature, 150 g of water was added and stirred violently for 10 minutes. Stirring was suspended and the solution was left to stand until phase separation occurred. Water which is the separated light phase was removed, and 1,162 g of an organic phase containing 1,1,2,3-tetrachloro-1-propene as the main component which is a heavy phase was obtained.

The distillation of 1,1,2,3-tetrachloro-1-propene was carried out to remove water. After water was removed, distillation was carried out at a maximum temperature of the bottom of the column of 125°C to obtain 1,065 g of 1,1,2,3-tetrachloro-1-propene having a purity of not less than 99.5 wt%. Not more than 0.1 wt% of 1,1,2,3-tetrachloro-1-propene was decomposed. About 87 g of the solution remaining at the bottom of the column was collected. The solution contained 10 g of 1,1,2,3-tetrachloro-1-propene and 40 g of 1,1,1,2,3-pentachloropropane. The bottom of the column after distillation was clean without a deposit.

The following experiment was conducted to create the state of the bottom of the column simulatively when distillation was carried out continuously for a long time and evaluate it. 50 g of the solution remaining at the bottom of the column after distillation was taken out and put into a 100 ml flask together with a SUS plate having a surface area of about 16 cm². After the solution was stirred at 130°C and about 15 kPa for 24 hours, the SUS plate was taken out and vacuum dried. As a result, it was confirmed that the weight of the SUS plate increased by 1.0 mg.

### Comparative Example 1

A reaction was carried out in the same manner as in Example 1. The conversion rate of 1,1,1,2,3-pentachloropropane was 96 % and the selectivity to 1,1,2,3-tetrachloro-1-propene was 98 %.

After the reaction, 1 ml of eugenol as a distillation stabilizer was added. Nitrogen was supplied to remove hydrogen chloride, and the concentration of hydrogen chloride was set to not more than 100 ppm.

1,160 g of the reaction solution was distilled at a maximum temperature of the bottom of the column of 125°C to obtain 1,062 g of 1,1,2,3-tetrachloro-1-propene having a purity of not less than 99.5 wt%. Not more than 0.1 wt% of 1,1,2,3-tetrachloro-1-propene was decomposed. A large amount of a deposit was observed at the bottom of the column after distillation.

Subsequently, 50 g of the solution remaining at the bottom of the column was taken out and put into a 100 ml flask together with a SUS plate having a surface area of about 16 cm² as in Example 1. After the solution was stirred at 130°C and about 15 kPa for 24 hours, the SUS plate was taken out and vacuum dried. As a result, the weight of the SUS plate increased by 40 mg and the surface of the SUS plate was covered with a large amount of a black deposit.

### Effect of the Invention

According to the present invention, distillation operation can be carried out continuously and stably for a long time when a reaction solution obtained by a dehydrochlorination reaction is purified by distillation. Further, according to the present invention, since a side reaction can be prevented even when a high distillation temperature and a high distillation pressure are set, the production method of the present invention is efficient from the viewpoints of distillation-related equipment cost, operation cost and time savings. Therefore, it is industrially useful.

## Claims

1. A chloroalkene production method comprising the steps of:
carrying out the dehydrochlorination reaction of a chloroalkane represented by the following formula (1) in the presence of a Lewis acid catalyst comprising a metal chloride to produce a chloroalkene represented by the following formula (2);
removing a metal component derived from the catalyst from the obtained reaction solution; and
distilling the reaction solution to obtain the chloroalkene.
Cₐ₊₁H_{b}Cl_{c+4} (1)
wherein "a" is an integer of 2 to 4, "b" is an integer of 1 to 2a, and "c" is an integer of 0 to (2a-1), with the proviso that (b+c) is 2a.
Cₐ₊₁H_{b-1}Cl_{c+3} (2)
wherein "a", "b" and "c" are as defined in the above formula (1).

2. The chloroalkene production method according to claim 1, wherein the metal component derived from the catalyst is removed by solvent extraction.

3. The chloroalkene production method according to claim 2, wherein the concentration of hydrogen chloride at the time of solvent extraction in the reaction solution obtained by the dehydrochlorination reaction is 1.0 x 10⁻⁵ to 5.0 x 10⁻² part by mass based on 1 part by mass of the chloroalkene represented by the formula (2).

4. The chloroalkene production method according to claim 2 or 3, wherein an extraction solvent used for solvent extraction is water or a polyhydric alcohol.

5. The chloroalkene production method according to claim 4, wherein the polyhydric alcohol is ethylene glycol, propylene glycol or glycerin.

6. The chloroalkene production method according to any one of claims 1 to 5, wherein the metal chloride is anhydrous aluminum chloride.

7. The chloroalkene production method according to any one of claims 1 to 6, wherein distillation is carried out at a temperature of the bottom of a distillation column of 70 to 250°C.

8. The chloroalkene production method according to any one of claims 1 to 7, wherein the concentration of the metal component derived from the residual catalyst in the reaction solution to be distilled is not more than 5.0 x 10⁻⁶ part by mass based on 1 part by mass of the chloroalkene represented by the above formula (2).

9. The chloroalkene production method according to any one of claims 1 to 8, wherein the chloroalkane represented by the above formula (1) is 1,1,1,3-tetrachloropropane and the chloroalkene represented by the above formula (2) is 1,1,3-trichloro-1-propene.

10. The chloroalkene production method according to any one of claims 1 to 9, wherein the chloroalkane represented by the above formula (1) is 1,1,1,2,3-pentachloropropane and the chloroalkene represented by the above formula (2) is 1,1,2,3-tetrachloro-1-propene.
